# EUROPÄISCHE PATENTSCHRIFT

(11) **EP 0 703 280 B1**
(45) Veröffentlichungstag und Bekanntmachung des Hinweises auf die Patenterteilung: **01.03.2000**
(21) Anmeldenummer: 95810568.6
(22) Anmeldetag: 14.09.1995
(51) Int. Cl.: C09B 47/067, C07D 487/22, G11B 7/24

(54) **Isomerengemische von alkoxysubstituierten Phtalocyaninen und Verfahren zu deren Herstellung**
Isomer mixtures of alkoxyphtalocyanines and process for their manufacture
Mélanges d'isomères de phtalocyanines substituées par des alcoxy et leur procédé de fabrication

(30) Priorität: 23.09.1994 CH 290694
(43) Veröffentlichungstag der Anmeldung: 27.03.1996
(73) Patentinhaber: Ciba Specialty Chemicals Holding Inc., 4057 Basel (CH)
(72) Erfinder: Wolleb, Heinz, Dr., CH-1723 Marly (CH)

(56) Entgegenhaltungen:
- EP-A- 0 337 209
- EP-A- 0 373 643
- EP-A- 0 492 508
- EP-A- 0 513 370
- US-H- H 477
- DATABASE WPI Section Ch, Week 9303 Derwent Publications Ltd., London, GB; Class E23, AN 93-021894 & JP-A-04 348 168 ( MITSUI TOATSU CHEM INC) , 3.Dezember 1992
- DATABASE WPI Section Ch, Week 8943 Derwent Publications Ltd., London, GB; Class A85, AN 89-314182 & JP-A-01 233 401 ( MITSUI TOATSU CHEM INC) , 19.September 1989

## Beschreibung

Die Erfindung betrifft ein Isomerengemisch von Alkoxy substituierten Phthalocyaninen sowie ein Verfahren zu ihrer Herstellung durch Umsetzung von Verbindungen der Formel V 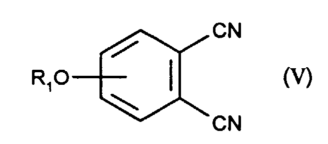 in Gegenwart eines Metallsalzes und einer Lewissäure, sowie Harnstoff und ausgewählter Lösungsmittel.

Die Verwendung von Farbstoffen, die im nahen Infrarotbereich (NIR-Bereich) Strahlung absorbieren, zur Aufzeichnung von Informationen in WORM-Systemen (write once read many), ist seit längerem bekannt und zum Beispiel von M. Emmelius et al. in Angewandte Chemie, Heft 11, Seiten 1475-1502 (1989) beschrieben. Durch die Laserbestrahlung in solchen Aufzeichnungsmaterialien kann die für die Aufzeichnung von Informationen in Form von Bits notwendige Änderung der Absorption durch physikalische Veränderungen (zum Beispiel durch Sublimation oder Diffusion) oder durch chemische Veränderungen (zum Beispiel Photochromie, Isomerisierungen oder thermische Zersetzung) erzielt werden.

Substituierte Phthalocyanine stellen eine wichtige Klasse von Farbstoffen für die Anwendung in solchen WORM-Systemen dar, da sie bei entsprechender peripherer Substitution, abhängig vom zentralen Metallatom, hohe NIR Absorptionen im Bereich von 700 nm bis 900 nm aufweisen können. Neben den Absorptionseigenschaften ist ihre Löslichkeit in organischen Lösungsmitteln, insbesondere aliphatischen Kohlenwasserstoffen, von grosser Bedeutung, da die Absorptionsschicht von optischen Speichermaterialien häufig in einem Schleuderbeschichtungs-Verfahren aus einem organischen Lösungsmittel hergestellt wird.

In der EP-A-337 209 werden mehrfach substituierte Alkylphthalocyanine beschrieben, die durch Umsetzung von substituierten Phthalodinitrilen in hochsiedenden Lösungsmitteln in Gegenwart einer Lewissäure erhalten werden können. Als Lösungsmittel werden Harnstoff, Chlornaphthalin, Nitrobenzol, Alkohole und Aminoalkohole vorgeschlagen. Die Reaktionstemperatur kann zum Beispiel 250°C betragen. Die Löslichkeit der erhaltenen Alkyl-Phthalocyanine in organischen Lösungsmitteln und die Zusammensetzung des Reaktionsproduktes werden nicht beschrieben.

Die Herstellung von Alkoxy substituierten Phthalocyaninen ist ebenfalls bekannt und die isomeren Tetra-isopropoxyphthalocyanine in Nouveau Journal de Chemie, Vol. 6, p. 653-658 (1982) beschrieben. Die Reaktion wird zum Beispiel an polymergebundenem Phthalodinitril durchgeführt, wobei man ein reines Isomeres erhält. Wird die Reaktion bei höherer Temperatur in Dimethylaminoethanol ohne Polymerbindung durchgeführt, entsteht ein Isomerengemisch unbekannter Verteilung.

Ein anderes Verfahren für die Herstellung von α-Alkoxy substituierten Phthalocyaninen wird zum Beispiel in EP-A-373643 und EP-A-492 508 beschrieben. EP-A-373643 beschreibt die Bildung eines einzigen symmetrischen Isomeren beim Erhitzen eines Gemisches von α-Alkoxy-phthalodinitril, Metallsalz, Base und Alkohol zum Rückfluss. In EP-A-492 508 dagegen legt man α-Alkoxy-phthalodinitril und eine organische Base in einem Alkohol bei 90-120°C vor und gibt bei dieser Temperatur ein Metallsalz zu, wobei man als Hauptprodukte 2 Stellungsisomere im Verhältnis 40/60 bis 60/40 erhält, von denen eines in organischen Lösungsmitteln gut löslich und das andere schlechter löslich ist. Alternativ kann nach EP-A-492 508 das entsprechende Diiminoisoindolin eingesetzt werden. Durch Variation der Basenzugabe und Temperatur kann das Isomerenverhältnis beeinflusst werden. Es entstehen jeweils 2 Isomere, von denen das besser lösliche im Überschuss von 85-95 Anteilen zu 5-15 Anteilen vorliegt. Diese Verfahrensvariante hat den Nachteil, dass man zuerst das Diiminoisoindolin herstellen muss und dadurch einen weiteren Reaktionsschritt benötigt. Weiterhin entsteht bei beiden in EP-A-492 508 beschriebenen Varianten überwiegend nur zwei der 4 möglichen Isomere, davon nur eines der zwei besser löslichen, was sich nachteilig auf das Assoziations- und Kristallisationsverhalten auswirkt.

In der Regel weist ein Gemisch mehrerer gut löslicher Isomere eine geringere Tendenz zu Assoziation und Kristallisation auf, als solche, worin ein einzelnes Isomer stark überwiegt oder worin wesentliche Mengen (z.B. ≧10%) geringlöslicher Isomere enthalten sind.

Die vorliegende Erfindung betrifft ein gut lösliches Isomerengemisch, enthaltend überwiegend zwei gut lösliche Stellungsisomere von Alkoxy substituierten Phthalocyaninen sowie ein Verfahren, bei dem a) gute Ausbeuten erzielt werden und b) unsymmetrische Stellungsisomere bevorzugt entstehen, wodurch eine gute Löslichkeit der α- oder β-Alkoxy substituierten Phthalocyanine in organischen Lösungsmitteln erreicht wird. Die Isomerengemische können auch als Zwischenprodukte für weitere Reaktionen dienen. So lassen sie sich zum Beispiel wie in der EP-A-513 370 beschrieben mit Halogen zu den entsprechenden halogenierten Alkoxy-Phthalocyaninen umsetzen, wobei eine Vielzahl von Isomeren entstehen kann, was für die Löslichkeit und die Lagerstabilität der Lösung vorteilhaft ist. In diesen halogenierten Isomerengemischen von Phthalocyaninen kann das Halogen noch weiter mit mindestens einem Phosphorsubstituenten ersetzt werden, wodurch sich die Polarität der Verbindungen und damit ihre Löslichkeit auf die verschiedensten Lösungsmittel anpassen lässt.

Ein Gegenstand der Erfindung ist ein Isomerengemisch α-Alkoxy substituierter Phthalocyanine der Formeln I-IV 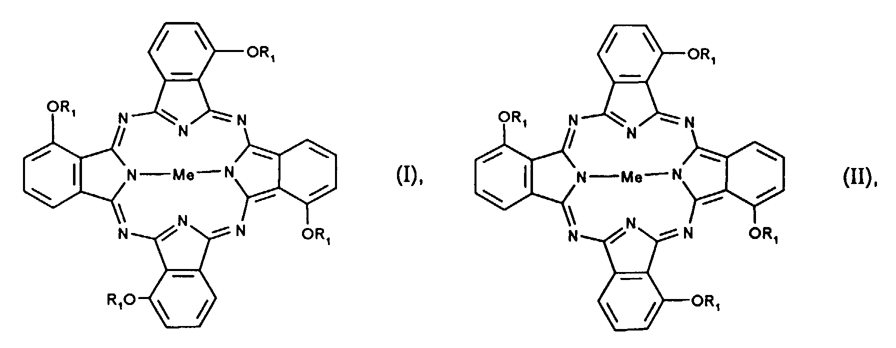 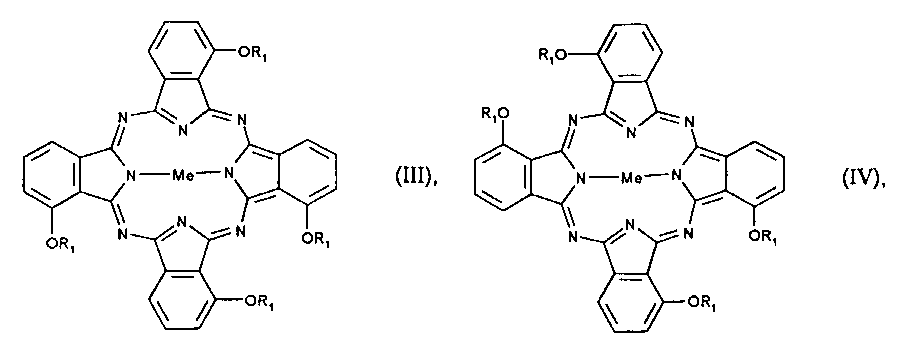 oder β-Alkoxy substituierter Phthalocyanine der Formeln Ia-IVa 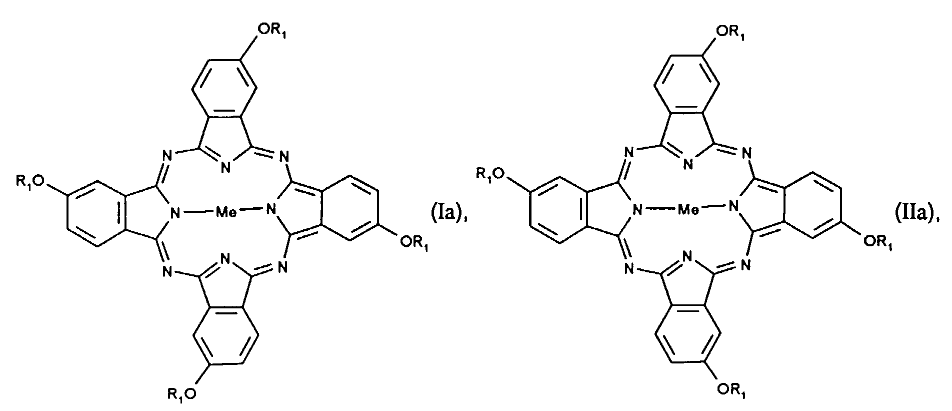 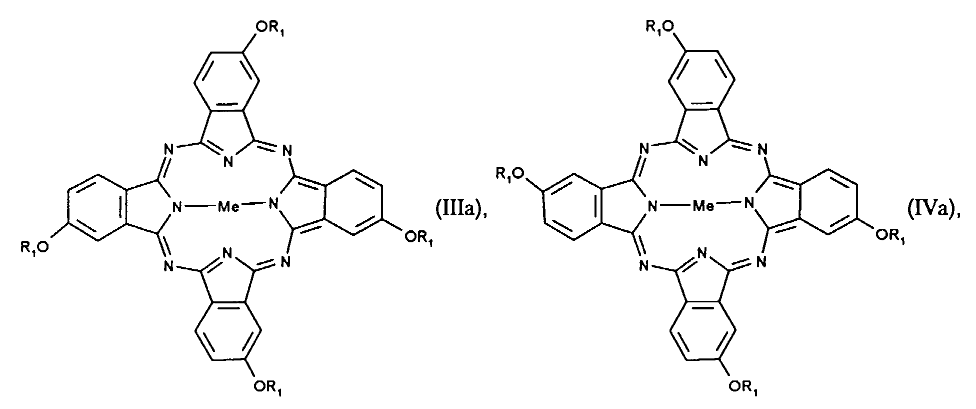 wobei
Me ein divalentes Metallatom oder ein divalentes Oxometall ist,
R₁ ein linearer oder verzweigter C₁-C₁₆-Alkyl-, C₃-C₁₆-Alkenyl- oder C₃-C₁₆-Alkinylrest ist, der unsubstituiert oder mit C₁-C₁₂-Alkoxy, -CN, NO₂, Halogen, -OH, Phenyl, Cyanophenyl, Nitrophenyl, Halogenphenyl, Hydroxyphenyl oder (C₁-C₁₂-Alkoxy)phenyl substituiert ist, das dadurch gekennzeichnet ist, dass die Isomeren der Formel II oder IIa und der Formel III oder IIIa zu mindestens 80% bezogen auf das gesamte Gemisch und die Isomeren der Formel I oder Ia und der Formel IV oder IVa zu höchstens 20% bezogen auf das gesamte Gemisch vorliegen, wobei das Verhältnis der Verbindungen der Formel II oder IIa zu Verbindungen der Formel III oder IIIa von 0,3 bis 3,0 zu 1 beträgt.

Prozentangaben bedeuten im Rahmen der vorliegenden Erfindung Gewichts %.

Bevorzugt sind Isomerengemische, bei denen die Isomeren der Formel II oder IIa und der Formel III oder IIIa zu mindestens 90% bezogen auf das gesamte Gemisch und die Isomeren der Formel I oder Ia und der Formel IV oder IVa zu höchstens 10% bezogen auf das gesamte Gemisch vorliegen.

Besonders bevorzugt sind Isomerengemische, bei denen die Isomeren der Formel II oder IIa und der Formel III oder IIIa zu mindestens 95% bezogen auf das gesamte Gemisch und die Isomeren der Formel I oder Ia und der Formel IV oder IVa zu höchstens 5% bezogen auf das gesamte Gemisch vorliegen.

Insbesondere sind Isomerengemische bevorzugt, worin das Verhältnis der Verbindungen der Formel II oder IIa zu Verbindungen der Formel III oder IIIa von 0,5 bis 2,0 zu 1 beträgt.

Beispiele für lineare oder verzweigte C₁-C₁₆-Alkylreste sind z.B. Methyl, Ethyl sowie die verschiedenen Stellungsisomeren von Propyl, Butyl, Pentyl, Hexyl, Heptyl, Octyl, Nonyl, Decyl, Undecyl, Dodecyl, Tridecyl, Tetradecyl, Pentadecyl oder Hexadecyl.

Bevorzugt sind C₄-C₁₂-Alkylreste.

Beispiele für C₃-C₁₆-Alkenylreste sind Propenyl, Butenyl, Pentenyl, Hexenyl, Heptenyl, Octenyl, Nonenyl, Decenyl, Undecenyl, Dodecenyl, Tridecenyl, Tetradecenyl, Pentadecenyl oder Hexadecenyl mit ihren verschiedenen Stellungsisomeren.

Bevorzugt sind C₄-C₁₂-Alkenylreste.

Beispiele für C₃-C₁₆-Alkinylreste sind Propinyl, Butinyl, Pentinyl, Hexinyl, Heptinyl, Octinyl, Noninyl, Decinyl, Undecinyl, Dodecinyl, Tridecinyl, Tetradecinyl, Pentadecinyl oder Hexadecinyl mit ihren verschiedenen Stellungsisomeren.

Bevorzugt sind C₄-C₁₂-Alkinylreste.

Bevorzugt sind die Alkyl-, Alkenyl- oder Alkinylreste verzweigt.

Halogen steht zum Beispiel für Fluor, Brom, Chlor oder Jod.

C₁-C₁₂-Alkoxy bedeutet zum Beispiel Methoxy, Ethoxy sowie die verschiedenen Stellungsisomeren von Propoxy, Butoxy, Pentoxy, Hexyloxy, Heptyloxy, Octyloxy, Nonyloxy, Decyloxy, Undecyloxy oder Dodecyloxy.

Bevorzugt sind C₁-C₈-Alkoxy.

Als zweiwertige Metallatome oder Oxometalle kommen eine Vielzahl von Metallen in Frage, wie dies zum Beispiel von F. H. Moser, A. L. Thomas in The Phthalocyanines, CRC Press 1983 beschrieben ist.

Bevorzugt stellt das divalente Metallatom oder Oxometall Cu(II), Zn(II), Fe(II), Ni(II), Ru(II), Rh(II), Pd(II), Pt(II), Mn(II), Mg(II), Be(II), Ca(II), Ba(II), Cd(II), Hg(II), Sn(II), Co(II), Pb(II) oder VO, MnO, TiO dar.

Besonders bevorzugt sind die divalenten Metallatome Zn(II), Sn(II), Cu(II), Ni(II), Co(II), Pb(II) oder Pd(II).

Ganz besonders bevorzugt sind die divalenten Metallatome Pd(II), Cu(II) oder Ni(II), insbesondere Cu(II) und speziell Pd(II).

In einer bevorzugten Untergruppe bedeutet R₁ einen linearen oder verzweigten C₁-C₁₆-Alkylrest, der unsubstituiert oder mit C₁-C₁₂-Alkoxy, -CN, NO₂, Halogen, -OH, Phenyl, Cyanophenyl, Nitrophenyl, Halogenphenyl, Hydroxyphenyl oder (C₁-C₁₂-Alkoxy)phenyl substituiert ist.

In einer besonders bevorzugten Untergruppe bedeutet R₁ einen linearen oder verzweigten C₄-C₁₂-Alkylrest, der unsubstituiert oder mit C₁-C₁₂-Alkoxy, -CN, NO₂, Halogen, -OH, Phenyl, Cyanophenyl, Nitrophenyl, Halogenphenyl, Hydroxyphenyl oder C₁-C₁₂ Alkoxyphenyl substituiert ist.

Ganz besonders bevorzugt steht R₁ für einen unsubstituierten linearen oder verzweigten C₄-C₁₂-Alkylrest, insbesondere einen verzweigten C₄-C₁₂-Alkylrest wie beispielsweise 2,4-Dimethyl-3-pentyloxy.

Die Substitution kann sowohl in β- als auch in α-Stellung erfolgen, bevorzugt steht der Substituent-OR₁ in α-Stellung.

Im Falle der α-Substitution können sich die 4 Stellungsisomeren der Formel I bis IV bei der Reaktion bilden. Bevorzugt sind Isomerengemische von Verbindungen der Formel I bis IV.

Die einzelnen Stellungsisomeren unterscheiden sich durch ihre Löslichkeit in organischen Lösungsmitteln, insbesondere in aliphatischen Kohlenwasserstoffen. Die hochsymmetrischen Isomere der Formel I und IV weisen die geringste Löslichkeit auf.

Im Falle der β-Substitution können die entsprechenden 4 Isomeren der Formel Ia bis IVa auftreten, wobei innerhalb dieser Reihe die Isomeren der Formel Ia und IVa ebenfalls die schlechtesten Löslichkeiten besitzen.

Ein weiterer Gegenstand der Erfindung ist ein Verfahren zur Herstellung eines Isomerengemisches von Verbindungen der Formel I bis IV oder Ia bis IVa, wobei die Isomeren der Formel II oder IIa und der Formel III oder IIIa zu mindestens 80% bezogen auf das gesamte Gemisch und die Isomeren der Formel I oder Ia und der Formel IV oder IVa zu höchstens 20% bezogen auf das gesamte Gemisch vorliegen, und das Verhältnis der Verbindungen der Formel II oder IIa zu Verbindungen der Formel III oder IIIa von 0,3 bis 3,0 zu 1 beträgt,
durch Umsetzung von Verbindungen der Formel V 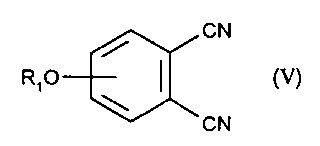 in Gegenwart eines Metallsalzes und einer Lewissäure, wobei R₁ die oben angegebene Bedeutung hat,
welches dadurch gekennzeichnet ist, dass die Reaktion in Gegenwart von Nitrobenzol, Nitrotoluol oder Nitroxylol und mindestens einer equimolaren Menge Harnstoff, bezogen auf die Verbindungen der Formel V, durchgeführt wird.

Bevorzugt werden Metallsalze eingesetzt bei denen das Anion von einer ein- oder zweibasigen anorganischen Säure, einer C₁-C₁₂-Carbonsäure oder einem C₅-C₁₂-β-Diketon stammt.

Als anorganische Säuren kommen vor allem HCl, HBr, H₂SO₄, HNO₃ oder HClO₄ in Frage. Geeignete C₁-C₁₂-Carbonsäuren sind zum Beispiel Ameisensäure, Essigsäure, Propionsäure, die verschiedenen Isomeren der Buttersäure, Valeriansäure oder Capronsäure. Als C₅-C₁₂-β-Diketone kommen zum Beispiel Acetylaceton, Hexan-2,4-Dion, Heptan-3,5-Dion, Heptan-2,4-Dion, die verschiedenen Stellungsisomeren Octan-, Nonan-, Decan-, Undecan- und Dodecan-β-Dione in Frage.

Besonders bevorzugt verwendet man als Metallsalz Pd(II)Cl₂, Cu(II)Cl₂, Zn(II)Cl₂, Ni(II)Cl₂, Cu(II)-acetylacetonat oder V(III)-acetylacetonat.

Ganz besonders bevorzugt werden Pd(II)Cl₂, Cu(II)Cl₂ oder Ni(II)Cl₂ eingesetzt.

Die Reaktion wird bevorzugt bei einem Molverhältnis der Verbindungen der Formel V zu Harnstoff von 1 : 1 bis 1: 20 durchgeführt und besonders bevorzugt in einem Molverhältnis von 1 : 1 bis 1 : 10 durchgeführt.

Das Gewichtsverhältnis von Harnstoff zu Nitrobenzol, Nitrotoluol oder Nitroxylol beträgt bevorzugt 1 : 1 bis 1 : 50 und besonders bevorzugt 1 : 5 bis 1 : 20.

Bevorzugt wird das Verfahren bei einer Temperatur von 130 - 250°C und besonders bevorzugt bei einer Temperatur von 130 - 190°C durchgeführt.

Die Druckbedingungen sind an sich unkritisch, es wird bevorzugt bei einem Druck von 1·10⁵ - 20·10⁵ Pa gearbeitet.

Je nach Metallatom kann die Reaktionszeit verschieden sein; bevorzugt beträgt sie von 0,5 bis 24 Stunden.

Lewissäuren sind dem Fachmann in grosser Zahl bekannt, wie zum Beispiel AlCl₃, AlBr₃, BF₃, BCl₃, SbCl₃, SbBr₃, AsBr₃, AsCl₃, ZnCl₂, ZnBr₂, SnCl₂, SnBr₂, Ammoniummolybdat oder Ammoniummolybdat-tetrahydrat und andere.

Bevorzugt wird als Lewissäure Ammoniummolybdat oder Ammoniummolybdat-tetrahydrat verwendet.

Die Lewissäure wird bevorzugt in einer Menge von 0,1 bis 5 Gew. % bezogen auf die Verbindungen der Formel V eingesetzt.

Ein anderer Gegenstand der Erfindung ist ein Material zur optischen Aufzeichnung und zur Speicherung von Informationen, bei dem auf einem transparenten und dielektrischen Träger mindestens eine Schicht eines erfindungsgemäss hergestellten Phthalocyanins der Formeln I-IV oder der Formeln Ia-IVa als Aufzeichnungsmaterial aufgebracht ist. Auf dem Material kann zusätzlich eine Reflexionsschicht und eine Schutzschicht aufgebracht sein. Im übrigen gelten z. B. die in der EP-A-546 994 beschriebenen Ausgestaltungen.

Das erfindungsgemässe Informationen enthaltende Material stellt insbesondere ein optisches Informationsmaterial vom WORM-Typ dar. Es kann zum Beispiel als abspielbare Compact Disc (CD), als Speichermaterial für Computer oder als Ausweis- und Sicherheitskarte verwendet werden.

Ein weiterer Gegenstand der Erfindung ist daher die Verwendung der Isomerengemische nach Anspruch 1 in optischen Speichern zur Informationsaufzeichnung in WORM-Systemen (write once read many).

Die nachfolgenden Beispiele erläutern die Erfindung.

Beispiel 1: In 200 ml Nitrobenzol werden 50 g (206 mMol) 3-(2,4-Dimethyl-3-pentyloxy)-phthalodinitril, 9,1 g (51,7 mMol) wasserfreies Palladiumchlorid, 24,8 g (413 mMol) Harnstoff und 1 g (2 Gew.%) Ammoniummolybdat vorgelegt und unter Rühren und Argonatmosphäre auf 160°C erwärmt. Anschliessend wird 4 Std bei dieser Temperatur gerührt, danach auf RT abgekühlt, mit Toluol verdünnt und über ein Filterhilfsmittel filtriert. Das Filtrat wird bei 100°C/10⁻¹mbar vollständig eingedampft. Der Rückstand wird in 400 ml Toluol aufgenommen und mit Toluol als Laufmittel über 500 g Kieselgel filtriert. Die Toluolphase wird auf 250 ml eingedampft und anschliessend auf 1,5 l Methanol getropft. Der Niederschlag wird abfiltriert und zweimal mit 100 ml Methanol gewaschen. Danach wird 12 Std bei 60°C/165 mbar getrocknet. Man erhält 32,5 g (59% d. Th.) eines grün-blauen Feststoffes mit einem λₘₐₓ, in N-Methylpyrrolidon (NMP) von 702 nm (ε = 215'190 l·mol⁻¹·cm⁻¹). Das NMR zeigt, dass die Isomeren I, II und III in einem Verhältnis von 5 zu 53 zu 42 vorliegen.

Beispiel 2: In 20 ml Nitrobenzol werden 5,0 g (20,6 mMol) 3-(2,4-Dimethyl-3-pentyloxy)-phthalodinitril, 1,35 g (5,15 mMol) Kupferacetylacetonat, 2,5 g (41,3 mMol) Harnstoff und 0,1 g (2 Gew.%) Ammoniummolybdat vorgelegt und unter Rühren und Argonatmosphäre auf 160°C erwärmt und anschliessend 4 Std bei dieser Temperatur gerührt. Anschliessend wird auf RT abgekühlt, mit Toluol verdünnt und durch ein Filterhilfsmittel filtriert. Das Filtrat wird bei 100°C/10⁻¹mbar vollständig eingedampft. Der Rückstand wird in Methylenchlorid aufgenommen, 100 g Kieselgel zugegeben, das Lösungsmittel eingedampft, danach auf eine Glasfilternutsche gegeben und das Produkt mit Hexan/Essigester 25:1 eluiert. Das Filtrat wird eingedampft, der Rückstand in 30 ml Toluol gelöst und auf 400 ml Methanol getropft. Der Niederschlag wird abfiltriert, zweimal mit 10 ml Methanol gewaschen und 12 Std bei 60°C/125 Torr getrocknet. Man erhält 2,0 g (38% d. Th.) eines grün-blauen Feststoffes mit einem λₘₐₓ (NMP) von 713 nm (ε = 220'140 l·mol⁻¹·cm⁻¹).

Beispiel 3: In 410 ml Nitrobenzol werden 100,0 g (0,41 Mol) 3-(2,4-Dimethyl-3-pentyloxy)-phthalodinitril, 14,0 g (0,1 Mol) Kupfer(II)chlorid, 49,6 g (0,82 Mol) Harnstoff und 2,0 g (2 Gew.%) Ammoniummolybdat vorgelegt und unter Rühren und Argonatmosphäre auf 160°C erwärmt und anschliessend 5 Std bei dieser Temperatur gerührt. Anschliessend wird auf RT abgekühlt, mit Toluol verdünnt und durch ein Filterhilfsmittel filtriert. Das Filtrat wird bei 10°C/10⁻¹Torr vollständig eingedampft. Der Rückstand wird in 1 l Toluol gelöst und mit Toluol als Laufmittel über 600 g Kieselgel filtriert. Das Filtat wird eingedampft, der Rückstand in 1,5 l Methanol aufgerührt, filtriert, mit Methanol gewaschen und über Nacht bei 60°C/165 mbar getrocknet. Man erhält 99,5 g (94% d. Th.) eines grün-blauen Feststoffes mit einem λₘₐₓ (NMP) von 712 nm (ε = 197'680 l·mol⁻¹·cm⁻¹).
Eine Dünnschichtchromatographie zeigt, dass die Isomeren I, II und III in einem Verhältnis von 5 zu 33 zu 62 vorliegen.

Beispiel 4 In 20 ml Nitrobenzol werden 5 g (20,6 mMol) 3-(2,4-Dimethyl-3-pentyloxy)-phthalodinitril, 1,79 g (5,15 mMol) Vanadium(III)acetylacetonat, 2,5 g (41,3 mMol) Harnstoff und 100 mg (2 Gew.%) Ammoniummolybdat vorgelegt, unter Rühren und Argonatmosphäre auf 150°C erwärmt und 4 Std bei dieser Temperatur gerührt. Danach wird auf RT abgekühlt, mit Toluol verdünnt und über ein Filterhilfsmittel filtriert. Das Filtrat wird vollständig eingedampft und mit Hexan/Essigester = 25:1 über 150 g Kieselgel filtriert. Die grüne Fraktion wird eingedampft, in 15 ml Toluol aufgenommen und auf 300 ml Methanol getropft. Der Niederschlag wird abfiltriert und über Nacht bei 60°C/165 mbar getrocknet.
Man erhält 1,9 g (36,1% d.Th.) eines grünen Feststoffes mit einem λₘₐₓ (NMP) von 743 nm (E = 180'860 l·mol⁻¹·cm⁻¹) und einem Gehalt an Vanadium von 4,52%.

Beispiel 5 In 60 ml Nitrobenzol werden 15 (61,9 mMol) 4-(2,4-Dimethyl-3-pentyloxy)-phthalodinitril, 2,74 g (15,48 mMol) Palladium(II)chlorid, 7,43 g (123,8 mMol) Harnstoff und 300 mg (2 Gew.%) Ammoniummolybdat vorgelegt, unter Rühren und Argonatmosphäre auf 160°C erwärmt und 24 Std. bei dieser Temperatur gerührt. Danach wird auf RT abgekühlt, mit Toluol verdünnt und über ein Filterhilfsmittel filtriert. Das Filtrat wird vollständig eingedampft und mit Toluol über 200 g Kieselgel filtriert. Die blaue Fraktion wird eingedampft, in 50 ml Toluol aufgenommen und auf 700 ml Methanol getropft. Der Niederschlag wird abfiltriert und über Nacht bei 60°C/165 mbar getrocknet.
Man erhält 8,0 g (48,0% d.Th.) eines blauen Feststoffes mit einem λₘₐₓ (NMP) von 675 nm.

Vergleichsbeispiel 1: 20 g Harnstoff werden zusammen mit 5,0 g (20,6 mMol) 3-(2,4-Di-methyl-3-pentyloxy)-phthalodinitril, 0,91 g (5,2 mMol) wasserfreiem Palladiumchlorid und 100 mg Ammoniummolybdat vorgelegt und unter Rühren und Argonatmosphäre 7 Std auf 160°C erhitzt. Es bildet sich zuerst eine braune Suspension, welche im Laufe der Reaktion fest wird. Anschliessend wird abgekühlt, der Feststoff in Methylenchlorid aufgenommen und filtriert. Danach werden 80 g Kieselgel zugegeben und das Lösungsmittel eingedampft. Das beladene Kieselgel wird auf eine Glasfilternutsche gegeben und das Produkt mit 1 l Hexan/Essigester 25:1 eluiert. Das Eluat wird eingedampft und der Rückstand in 15 ml Toluol gelöst und auf 300 ml Methanol getropft. Der Niederschlag wird abfiltriert und zweimal mit 10 ml Methanol gewaschen. Danach wird 12 Std bei 60°C/165 mbar getrocknet. Man erhält 1,2 g (21,6% d. Th.) eines blau-grünen Pulvers mit einem λₘₐₓ (NMP) von 702 nm (ε = 195'780 l·mol⁻¹·cm⁻¹).

Vergleichsbeispiel 2: In 20 ml Nitrobenzol werden 5,0 g (20,6 mMol) 3-(2,4-Dimethyl-3-pentyloxy)-phthalodinitril, 1,09 g (6,2 mMol) wasserfreies Palladiumchlorid und 100 mg Ammoniummolybdat vorgelegt und unter Rühren und Argonatmosphäre 4 Std auf 160°C erhitzt. Anschliessend wird abgekühlt, filtriert, gut mit Toluol gewaschen und das Filtrat bei 110°C am Hochvakuum vollständig eingedampft. Das Rohprodukt wird in Methylenchlorid gelöst, mit 70 g Kieselgel versetzt und das Lösungsmittel eingedampft. Das beladene Kieselgel wird auf eine Glasfilternutsche gegeben und das Produkt mit Hexan/Essigester 25:1 eluiert. Das Eluat wird eingedampft und der Rückstand bei 60°C/165 mbar 12 Std getrocknet. Man erhält 0,80 g (14%) eines blau-grünen Pulvers mit einem λₘₐₓ (NMP) von 702 nm (ε = 225'080 l·mol⁻¹·cm⁻¹).

## Patentansprüche

1. Isomerengemisch α-Alkoxy substituierter Phthalocyanine der Formeln I-IV 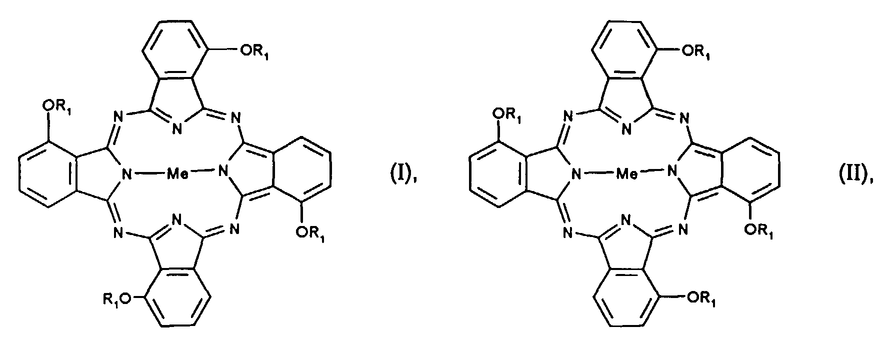 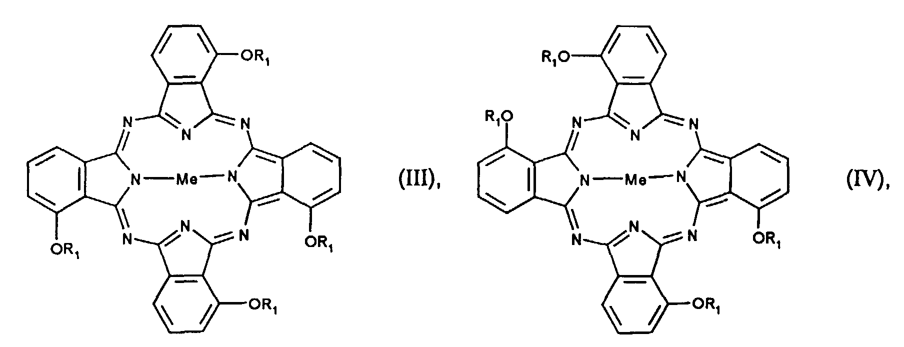 oder β-Alkoxy substituierter Phthalocyanine der Formeln Ia-IVa 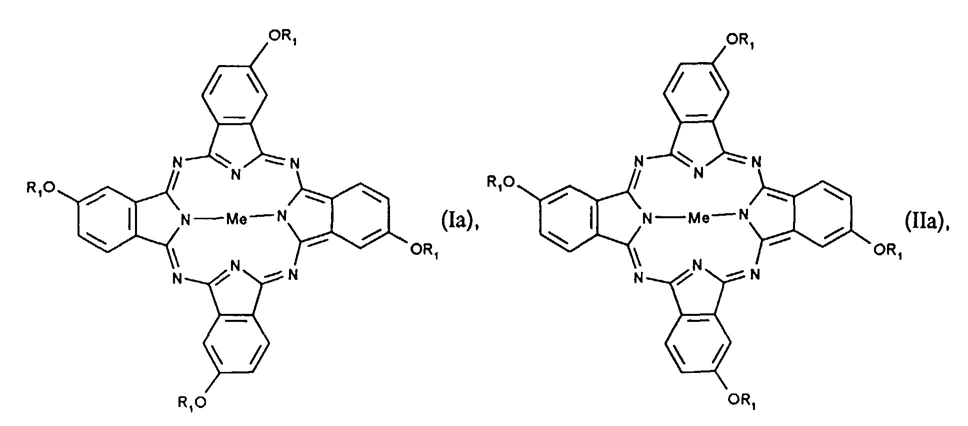 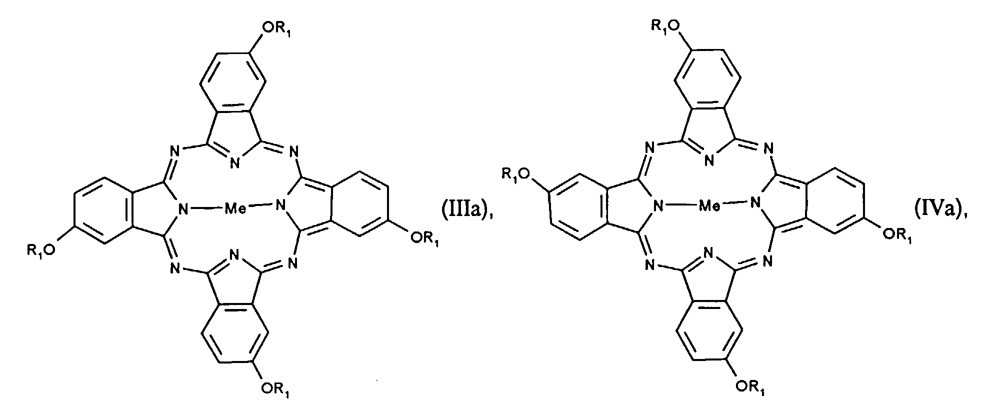 wobei
Me ein divalentes Metallatom oder ein divalentes Oxometall ist,
R₁ ein linearer oder verzweigter C₁-C₁₆-Alkyl-, C₃-C₁₆-Alkenyl- oder C₃-C₁₆-Alkinylrest ist, der unsubstituiert oder mit C₁-C₁₂-Alkoxy, -CN, NO₂, Halogen, -OH, Phenyl, Cyanophenyl, Nitrophenyl, Halogenphenyl, Hydroxyphenyl oder (C₁-C₁₂-Alkoxy)phenyl substituiert ist,
dadurch gekennzeichnet, dass die Isomeren der Formel II oder IIa und der Formel III oder IIIa zu mindestens 80% bezogen auf das gesamte Gemisch und die Isomeren der Formel I oder Ia und der Formel IV oder IVa zu höchstens 20% bezogen auf das gesamte Gemisch vorliegen, wobei das Verhältnis der Verbindungen der Formel II oder IIa zu Verbindungen der Formel III oder IIIa von 0,3 bis 3,0 zu 1 beträgt.

2. Isomerengemisch nach Anspruch 1, dadurch gekennzeichnet, dass die Isomeren der Formel II oder IIa und der Formel III oder IIIa zu mindestens 90%, bevorzugt mindestens 95%, bezogen auf das gesamte Gemisch und die Isomeren der Formel I oder Ia und der Formel IV oder IVa zu höchstens 10%, bevorzugt höchstens 5%, bezogen auf das gesamte Gemisch vorliegen.

3. Isomerengemisch nach Anspruch 1, dadurch gekennzeichnet, dass das divalente Metallatom oder Oxometall Me Cu(II), Zn(II), Fe(II), Ni(II), Ru(II), Rh(II), Pd(II), Pt(II), Mn(II), Mg(II), Be(II), Ca(II), Ba(II), Cd(II), Hg(II), Sn(II), Co(II), Pb(II) oder VO, MnO, TiO, bevorzugt Zn(II), Sn(II), Cu(II), Ni(II), Co(II), Pb(II) oder Pd(II), besonders bevorzugt Cu(II) oder Pd(II), ist.

4. Isomerengemisch nach Anspruch 1, dadurch gekennzeichnet, dass R₁ einen linearen oder verzweigten C₁-C₁₆-Alkylrest, der unsubstituiert oder mit C₁-C₁₂-Alkoxy, -CN, NO₂, Halogen, -OH, Phenyl, Cyanophenyl, Nitrophenyl, Halogenphenyl, Hydroxyphenyl oder (C₁-C₁₂-Alkoxy)phenyl substituiert ist, bevorzugt einen linearen oder verzweigten C₄-C₁₂-Alkylrest, der mit C₁-C₁₂-Alkoxy, -CN, NO₂, Halogen, -OH, Phenyl, Cyanophenyl, Nitrophenyl, Halogenphenyl, Hydroxyphenyl oder (C₁-C₁₂-Alkoxy)phenyl substituiert oder besonders bevorzugt unsubstituiert ist, darstellt.

5. Isomerengemische nach Anspruch 1, dadurch gekennzeichnet, dass die Verbindungen der Formel I bis IV enthalten sind.

6. Verfahren zur Herstellung eines Isomerengemisches nach Anspruch 1, durch Umsetzung von Verbindungen der Formel V 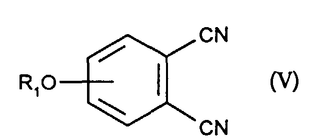 in Gegenwart eines Metallsalzes und einer Lewissäure,
wobei R₁ die im Anspruch 1 angegebene Bedeutung hat,
dadurch gekennzeichnet, dass die Reaktion in Gegenwart von Nitrobenzol, Nitrotoluol oder Nitroxylol und einer mindestens equimolaren Menge Harnstoff, bezogen auf die Verbindungen der Formel V, durchgeführt wird.

7. Verfahren nach Anspruch 6, dadurch gekennzeichnet, dass das Metallsalz das Anion einer ein- oder zweibasigen Mineralsäure, C₁-C₁₂-Carbonsäure oder eines C₅-C₁₂-β-Diketons enthält.

8. Verfahren nach Anspruch 6, dadurch gekennzeichnet, dass das Metallsalz Pd(II)Cl₂, Cu(II)Cl₂, Zn(II)Cl₂, Ni(II)Cl₂, Cu(II)-acetylacetonat oder V(III)-acetylacetonat, bevorzugt Pd(II)Cl₂, Cu(II)Cl₂ oder Ni(II)Cl₂, ist.

9. Verfahren nach Anspruch 6, dadurch gekennzeichnet, dass die Reaktion bei einem Molverhältnis der Verbindungen der Formel V zu Harnstoff von 1 : 1 bis 1 : 20 durchgeführt wird.

10. Verfahren nach Anspruch 6, dadurch gekennzeichnet, dass das Gewichtsverhältnis von Harnstoff zu Nitrobenzol, Nitrotoluol oder Nitroxylol 1 : 1 bis 1: 50, bevorzugt 5 bis 1 : 1: 20 beträgt.

11. Verfahren nach Anspruch 6, dadurch gekennzeichnet, dass es bei einer Temperatur von 130 - 250°C,bevorzugt von 130 - 190°C, durchgeführt wird.

12. Verfahren nach Anspruch 6, dadurch gekennzeichnet, dass es bei einem Druck von 1.10⁵ - 20.10⁵ Pa durchgeführt wird.

13. Verfahren nach Anspruch 6, dadurch gekennzeichnet, dass die Lewissäure Ammoniummolybdat oder Ammoniummolybdat-tetrahydrat ist.

14. Verfahren nach Anspruch 6, dadurch gekennzeichnet, dass die Lewissäure in einer Menge von 0,1 bis 5 Gew. % bezogen auf die Verbindungen der Formel V vorliegt.

15. Material zur optischen Aufzeichnung und zur Speicherung von Informationen, bei dem auf einem transparenten und dielektrischen Träger mindestens eine Schicht eines Isomerengemisches nach Anspruch 1 aufgebracht ist.

16. Verwendung der Isomerengemische nach Anspruch 1 in optischen Speichern zur Informationsaufzeichnung in WORM-Systemen (write once read many).

## Claims

1. A mixture of isomeric α-alkoxy-substituted phthalocyanines of the formulae I-IV 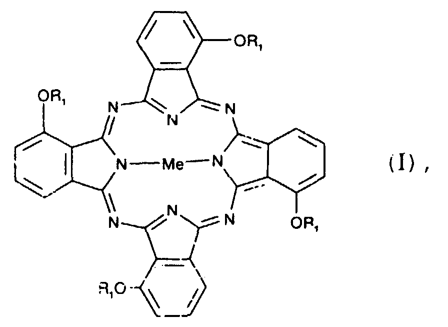 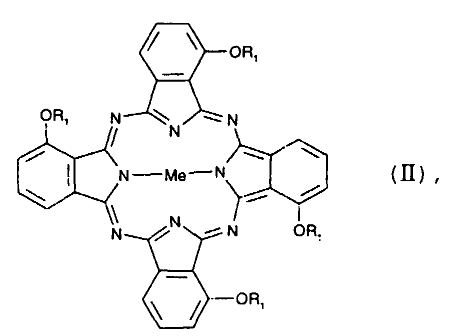 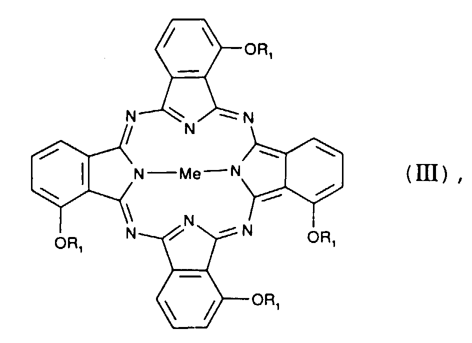 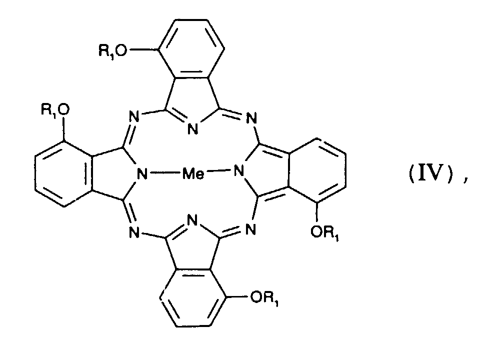 or β-alkoxy-substituted phthalocyanines of the formulae Ia-IVa 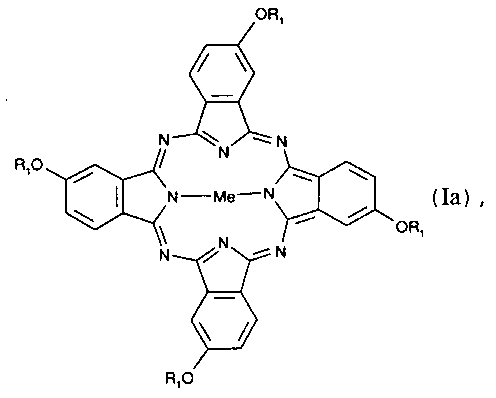 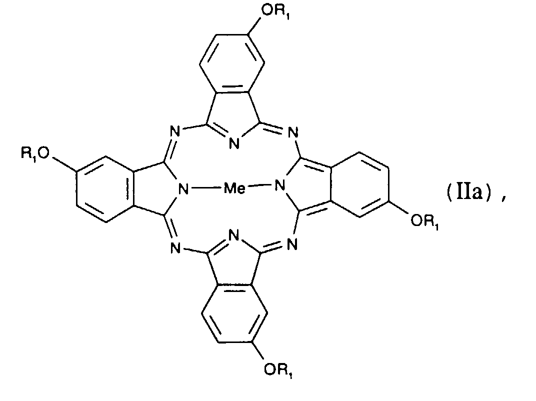 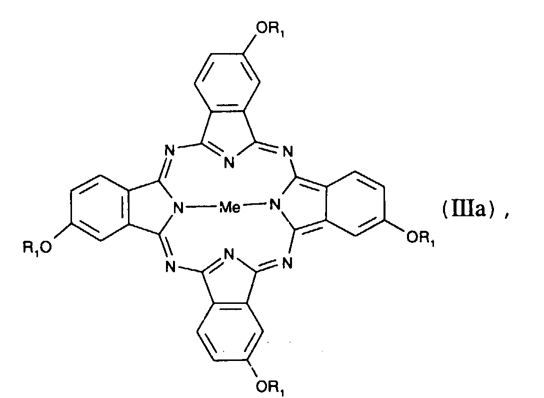 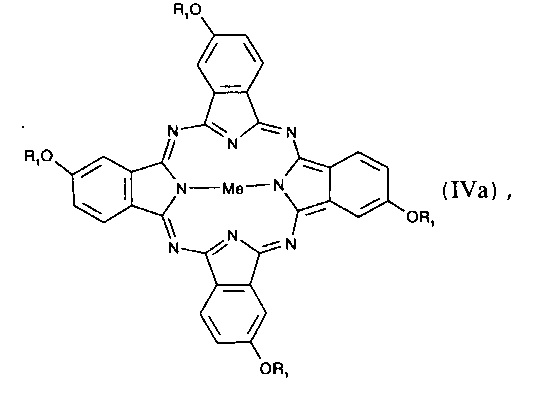 where
Me is a divalent metal atom or a divalent oxo metal,
R₁ is a linear or branched C₁-C₁₆alkyl, C₃-C₁₆alkenyl or C₃-C₁₆alkynyl radical, which is unsubstituted or substituted by C₁-C₁₂alkoxy, -CN, NO₂, halogen, -OH, phenyl, cyanophenyl, nitrophenyl, halophenyl, hydroxyphenyl or (C₁-C₁₂alkoxy)phenyl,
wherein the isomers of the formula II or IIa and of the formula III or IIIa make up at least 80 % of the total mixture, and the isomers of the formula I or Ia and of the formula IV or IVa make up at most 20 % of the total mixture, with the ratio between the compounds of the formula II or IIa and the compounds of the formula III or IIIa being from 0.3 to 3.0 : 1.

2. An isomer mixture according to claim 1, wherein the isomers of the formula II or IIa and of the formula III or IIIa make up at least 90 %, preferably at least 95 %, of the total mixture and the isomers of the formula I or Ia and of the formula IV or IVa make up at most 10 %, preferably at most 5 %, of the total mixture.

3. An isomer mixture according to claim 1, wherein the divalent metal atom or oxo metal Me is Cu(II), Zn(II), Fe(II), Ni(II), Ru(II), Rh(II), Pd(II), Pt(II), Mn(II), Mg(II), Be(II), Ca(II), Ba(II), Cd(II), Hg(II), Sn(II), Co(II), Pb(II) or VO, MnO, TiO, preferably Zn(II), Sn(II), Cu(II), Ni(II), Co(II), Pb(II) or Pd(II), particularly preferably Cu(II) or Pd(II).

4. An isomer mixture according to claim 1, wherein R₁ is a linear or branched C₁-C₁₆alkyl radical which is unsubstituted or substituted by C₁-C₁₂alkoxy, -CN, NO₂, halogen, -OH, phenyl, cyanophenyl, nitrophenyl, halophenyl, hydroxyphenyl or (C₁-C₁₂alkoxy)phenyl, preferably a linear or branched C₄-C₁₂alkyl radical which is substituted by C₁-C₁₂alkoxy, -CN, NO₂, halogen, -OH, phenyl, cyanophenyl, nitrophenyl, halophenyl, hydroxyphenyl or (C₁-C₁₂alkoxy)phenyl or particularly preferably is unsubstituted.

5. An isomer mixture according to claim 1, which comprises the compounds of the formulae I to IV.

6. A process for the preparation of an isomer mixture according to claim 1 by reacting compounds of the formula V 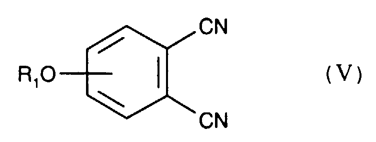 in the presence of a metal salt and a Lewis acid, where R₁ is as defined in claim 1,
which comprises carrying out the reaction in the presence of nitrobenzene, nitrotoluene or nitroxylene and an at least equimolar amount of urea, based on the compounds of the formula V.

7. A process according to claim 6, wherein the metal salt contains the anion of a monobasic or dibasic mineral acid, C₁-C₁₂carboxylic acid or C₅-C₁₂-β-diketone.

8. A process according to claim 6, wherein the metal salt is Pd(II)Cl₂, Cu(II)Cl₂, Zn(II)Cl₂, Ni(II)Cl₂, Cu(II) acetylacetonate or V(III) acetylacetonate, preferably Pd(II)Cl₂, Cu(II)Cl₂ or Ni(II)Cl₂.

9. A process according to claim 6, wherein the reaction is carried out at a molar ratio between the compounds of the formula V and urea of from 1 : 1 to 1 : 20.

10. A process according to claim 6, wherein the weight ratio between urea and nitrobenzene, nitrotoluene or nitroxylene is from 1 : 1 to 1 : 50, preferably from 1 : 5 to 1 : 20.

11. A process according to claim 6, which is carried out at a temperature of from 130 to 250°C, preferably from 130 to 190°C.

12. A process according to claim 6, which is carried out at a pressure of from 1·10⁵ to 20·10⁵ Pa.

13. A process according to claim 6, wherein the Lewis acid is ammonium molybdate or ammonium molybdate tetrahydrate.

14. A process according to claim 6, wherein the Lewis acid is present in an amount of from 0.1 to 5 % by weight, based on the compounds of the formula V.

15. A material for the optical recording and storage of information in which at least one layer of an isomer mixture according to claim 1 has been applied to a transparent, dielectric carrier.

16. The use of an isomer mixture according to claim 1 in optical storage media for information recording in WORM (write once read many) systems.

## Revendications

1. Mélange de phtalocyanines substituées par des substituants α-alkoxy de formules I-IV 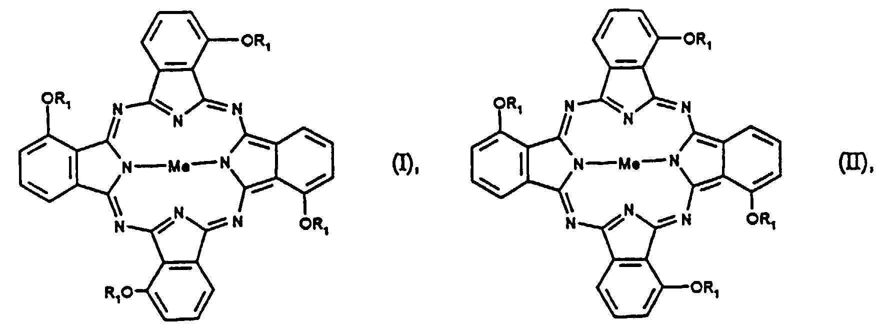 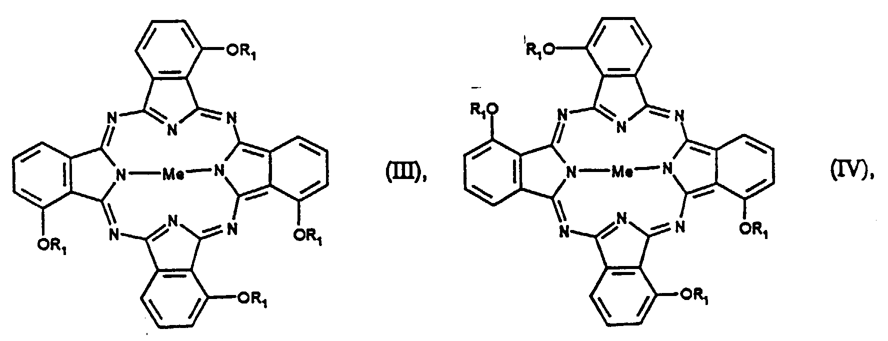 ou de phtalocyanines substituées par des substituants β-alkoxy de formules Ia-IVa 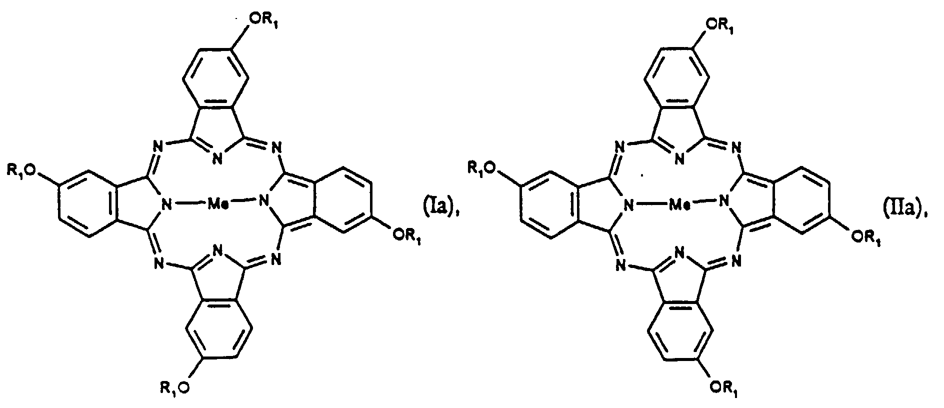 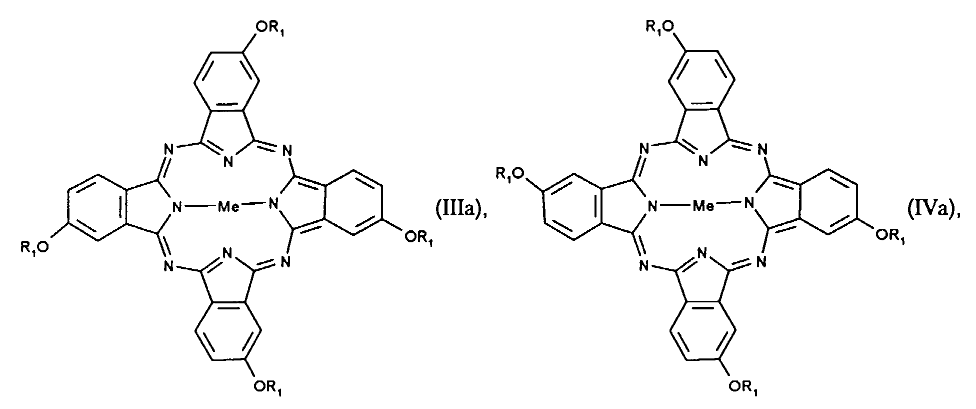 où
Me représente un atome métallique bivalent ou un oxométal bivalent,
R₁ représente un reste alkyle en C₁-C₁₆, alcényle en C₃-C₁₆ ou alcynyle en C₃-C₁₆ à chaîne droite ou ramifiée, non substitué ou substitué par des substituants alkoxy en C₁-C₁₂, -CN, NO₂, halogène, -OH, phényle, cyanophényle, nitrophényle, halophényle, hydroxyphényle ou (alkoxy en C₁-C₁₂)phényle,
caractérisé en ce que les isomères de formule II ou IIa et de formule III ou IIIa sont présents en une quantité d'au moins 80% par rapport à la totalité du mélange et les isomères de formule I ou Ia et de formule IV ou IVa sont présents en une quantité de 20% au maximum par rapport à la totalité du mélange, le rapport des composés de formule II ou IIa aux composés de formule III ou IIIa étant de 0,3 à 3,0 à 1.

2. Mélange d'isomères selon la revendication 1, caractérisé en ce que les isomères de formule II ou IIa et de formule III ou IIIa sont présents en une quantité d'au moins 90%, de préférence de 95%, par rapport à la totalité du mélange, et les isomères de formule I ou Ia et de formule IV ou IVa sont présents en une quantité de 10% au maximum, de préférence de 5% au maximum, par rapport à la totalité du mélange.

3. Mélange d'isomères selon la revendication 1, caractérisé en ce que l'oxométal ou d'atome de métal bivalents Me représente Cu(II), Zn(II), Fe(II), Ni(II), Ru(II), Rh(II), Pd(II), Pt(II), Mn(II), Mg(II), Be(II), Ca(II), Cd(II), Hg(II), Sn(II), Co(II), Pb(II) ou VO, MnO, TiO, de préférence Zn(II), Sn(II), Cu(II), Ni(II), Co(II), Pb(II) ou Pd(II), de manière particulièrement préférée Cu(II) ou Pd(II).

4. Mélange d'isomères selon la revendication 1, caractérisé en ce que R₁ représente reste alkyle en C₁-C₁₆ à chaîne droite ou ramifiée, lequel est non substitué ou substitué par des substituants alkoxy en C₁-C₁₂, -CN, NO₂, halogène, -OH, phényle, cyanophényle, nitrophényle, halophényle, hydroxyphényle ou (alkoxy en C₁-C₁₂)phényle, de préférence un reste alkyle en C₄-C₁₂ à chaîne droite ou ramifiée, lequel est substitué par des substituants alkoxy en C₁-C₁₂, -CN, NO₂, halogène, -OH, phényle, cyanophényle, nitrophényle, halophényle, hydroxyphényle ou (alkoxy en C₁-C₁₂)phényle ou de manière particulièrement préfére, non substitué.

5. Mélange d'isomères selon la revendication 1, caractérisé en ce qu'il contient les composés de formule I à IV.

6. Procédé pour la préparation d'un mélange d'isomères selon la revendication 1, par réaction de composés de formule V 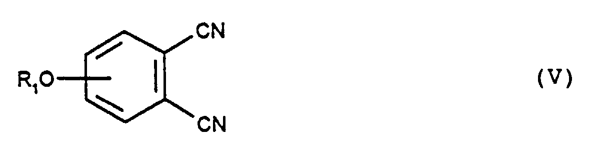 en présence d'un sel métallique ou d'un acide de Lewis, R₁ possédant la signification donnée à la revendication 1,
caractérisé en ce que l'on met en oeuvre la réaction en présence du nitrobenzène, du nitrotoluène ou nitroxylène et d'au moins une quantité équimolaire d'urée, par rapport aux composés de formule V.

7. Procédé selon la revendication 6, caractérisé en ce que le sel métallique contient l'anion d'un acide minérale mono- ou bifonctionnel, d'un acide carboxylique en C₁-C₁₂ ou d'une β-dicétone en C₅-C₁₂.

8. Procédé selon la revendication 6, caractérisé en ce que le sel métallique est Pd(II)Cl₂, Cu(II)Cl₂, Zn(II)Cl₂, Ni(II)Cl₂, l'acétylacétonate de Cu(II) ou l'acétylacétonate de V(III), de préférence Pd(II)Cl₂, Cu(II)Cl₂ ou Ni(II)Cl₂.

9. Procédé selon la revendication 6, caractérisé en ce que la réaction est mise en oeuvre avec un rapport molaire des composés de formule V à l'urée de 1:1 à 1:20.

10. Procédé selon la revendication 6, caractérisé en ce que le rapport pondéral de l'urée au nitrobenzène, au nitrotoluène ou nitroxylène est de 1:1 à 1:50, de préférence de 1:5 à 1:20.

11. Procédé selon la revendication 6, caractérisé en ce qu'il est mis en oeuvre à une température de 130 à 250°C, de préférence de 130 à 190°C.

12. Procédé selon la revendication 6, caractérisé en ce qu'il est mis à l'oeuvre sous une pression de 1·10⁵ à 20·10⁵ Pa.

13. Procédé selon la revendication 6, caractérisé en ce que l'acide de Lewis est le molybdate d'ammonium ou le molybdate d'ammonium tétrahydraté.

14. Procédé selon la revendication 6, caractérisé en ce que l'acide de Lewis est présent en une quantité de 0,1 à 5% en masse par rapport à la totalité des composés de formule V.

15. Matière pour l'enregistrement optique et pour le stockage d'informations où l'on applique sur un support transparent et diélectrique au moins une couche d'un mélange d'isomères selon la revendication 1.

16. Utilisation des mélanges d'isomères selon la revendication 1 dans des mémoires optiques pour l'enregistrement d'informations en systèmes WORMS (write once read many).
